# EUROPEAN PATENT APPLICATION

(11) **EP 4 617 353 A1**
(43) Date of publication of application: **17.09.2025**
(21) Application number: 24163599.4
(22) Date of filing: 14.03.2024
(51) Int. Cl.: C12M 1/26, C12M 1/36, G05B 13/04, G05B 17/02

(54) **METHOD FOR CONTROLLING A BIOPROCESS**

(71) Applicant: Sartorius Stedim Biotech GmbH, 37079 Göttingen (DE)
(72) Inventor: Scholz, Jochen, 37077 Göttingen (DE); Grimm, Christian, 37308 Heilbad Heiligenstadt (DE); Schmidberger, Timo, 83098 Brannenburg (DE)
(74) Representative: Gottschald Patentanwälte Partnerschaft mbB

(57) **Abstract**

The invention relates to a method for controlling a bioprocess (1), wherein in a model control routine (5) a bioprocess control unit (4) controls at least one process parameter (6) of the bioprocess (1) according to a model-based control loop (7). It is proposed that during the model control routine (5) the bioprocess control unit (4) repeatedly performs a first evaluation (13) of the model-based control loop (7), that if a first decision criterion is fulfilled, the bioprocess control unit (4) switches from the model control routine (5) to a sample control routine (15), that in the sample control routine (15) the bioprocess control unit (4) controls the bioprocess (1) according to a sample-based control loop (23), that at least one sample input parameter (24) of the sample-based control loop (23) is derived from a measurement value.

## Description

The present invention relates to a method for controlling a bioprocess according to the general part of claim 1, to a bioprocess control unit adapted for use in the proposed method according to claim 13 and to a bioprocess arrangement adapted for use in the proposed method according to claim 14.

The term "bioprocess" presently represents any kind of biotechnological process, in particular biopharmaceutical process. An example of such bioprocess is the use of a bioreactor to cultivate microorganisms or mammalian cells under given conditions. There is a strong need to industrialize bioprocesses by increasing the efficiency and decreasing the need for resources. At the same time, it is essential for many bioprocesses that they do not run out of predefined bounds. Many bioproducts are very costly and losing a batch due to a failed process would be severe. However, different from e.g. the chemical industry, the variance between batches of the same process can be significant as the basis of the process is a biological organism. It is currently the case that many critical process parameters and critical quality attributes are measured atline or offline, either to retrospectively evaluate a process (mainly offline) or to control a process (atline).

Challenging is that atline measurements of critical process parameters and critical quality attributes are slow, costly and may consume a significant amount of bioproduct for the measurement if used for controlling the bioprocess. Model-based control of bioprocesses is known and used to overcome the disadvantages of sampling, however, may lead to a reduced certainty. If the model deviates from reality, the bioproduct may have to be completely discarded when the deviation is detected from offline measurements too late.

The invention is based on the problem of improving the known control method such that a more cost-efficient control with an increased certainty of keeping the critical quality attributes in defined bounds.

The above-noted object is solved by the features of the characterizing part of claim 1.

The invention relates to a method for controlling a bioprocess, wherein the bioprocess runs in a biocontainer of a bioprocess arrangement, wherein the bioprocess arrangement comprises a bioprocess control unit, wherein in a model control routine the bioprocess control unit controls at least one process parameter of the bioprocess according to a model-based control loop, wherein at least one model input parameter of the model-based control loop is derived from a model prediction.

The main realization of the present invention is that a model-based control loop is generally advantageous as data points for the control loop (sampled data points or model-derived data points) can be obtained with a higher frequency compared to atline measurements. However, the model output is only considered valid if certain specific statistical checkpoints or other quality indicators are met. In case those checkpoints are not met, the mitigation foresees to switch to the atline sampling for control. Once the statistical checkpoints are met again, the model-based control may become active again. It is proposed to combine a model-based control and a sample-based control by evaluating the quality of the model and switching to the sample-based control if the quality of the model is not high enough. Naturally, the model needs to be of a high quality, but another layer of security is added by double-checking the model output via sampling and if the model deviates from reality, using the samples for controlling the bioprocess.

In detail, it is proposed that during the model control routine the bioprocess control unit repeatedly performs a first evaluation of the model-based control loop thereby checking whether a first decision criterion is fulfilled, that if the first decision criterion is fulfilled, the bioprocess control unit switches from the model control routine to a sample control routine, that the bioprocess arrangement comprises a sampling arrangement for drawing a sample from the biocontainer, that the bioprocess arrangement comprises an analysis arrangement, that the analysis arrangement comprises a, in particular atline, analysis device, that in the sample control routine the analysis device analyses samples provided by the sampling arrangement from the biocontainer to determine a measurement value, that in the sample control routine the bioprocess control unit controls the bioprocess according to a sample-based control loop, at least one sample input parameter of the sample-based control loop is derived from the measurement value.

The sampling may be done to determine any value which helps with controlling the bioprocess. In one embodiment according to claim 2, the measurement value and/or the sample input parameter derived from the measurement value correlates with or is the model input parameter and/or the controlled process parameter. In particular, a critical quality attribute, critical process parameter or key performance indicator of the bioprocess may be measured directly to get improved knowledge about the current state of the bioprocess regarding its critical parameters and to then get the bioprocess back on track based on this improved knowledge, if the bioprocess has diverted from a predefined path.

In an embodiment according to claim 3, the model is evaluated regarding its applicability. If the model deviates from reality, the predictions of the model may not be good enough, so that the sample-based control is started.

Claims 4 and 5 relate to switching back from the sample-based control to the model-based control based on a second evaluation of the model. After the bioprocess is back in a condition in which the model is considered applicable, the model can be used again for controlling the bioprocess.

One reason for the model deviating from reality may be a sensor drift. The measurement values can therefore be used to recalibrate a sensor according to claim 6.

In some embodiments according to claim 7, the first evaluation of the model may be performed time-based. The second evaluation may be performed at least between each drawing of a sample, thereby making it possible to draw the least necessary number of samples. Both evaluations may be substantially the same, though with different thresholds as mentioned in claim 5.

According to claim 8, the first evaluation may also be triggered event-based, e.g. at certain for example critical stages of the bioprocess the model may be evaluated. It may also be the case that the model is allowed less deviation at certain critical stages.

While the sample is analyzed, the bioprocess may still be controlled according to the model. However, the product produced at least in this period may be flagged to make sure that no bad product is released if the model turns out to have deviated too far. If the product can't be separated, this flag may concern all of the product.

The sampling may be automatic according to claim 10. The model may use data from inline and/or online sensors for the predictions (claim 11). The bioprocess control unit may control several process parameters according to several control loops and switch only some as described in claim 12.

Another teaching according to claim 13, which is of equal importance, relates to a bioprocess control unit adapted for use in the method according to one of the preceding claims.

All explanations given with regard to the proposed method are fully applicable.

Another teaching according to claim 14, which is of equal importance, relates to a bioprocess arrangement adapted for use in the proposed method, comprising a proposed bioprocess control unit.

All explanations given with regard to the proposed method and the proposed bioprocess control unit are fully applicable.

In the following, embodiments of the invention are explained with respect to the drawing. The drawing shows in
- Fig. 1,: a bioprocess arrangement,
- Fig. 2,: in a) a control of the bioprocess with a model-based control loop and in b) switched to a sample-based control loop and
- Fig. 3,: a flowchart of the proposed method.

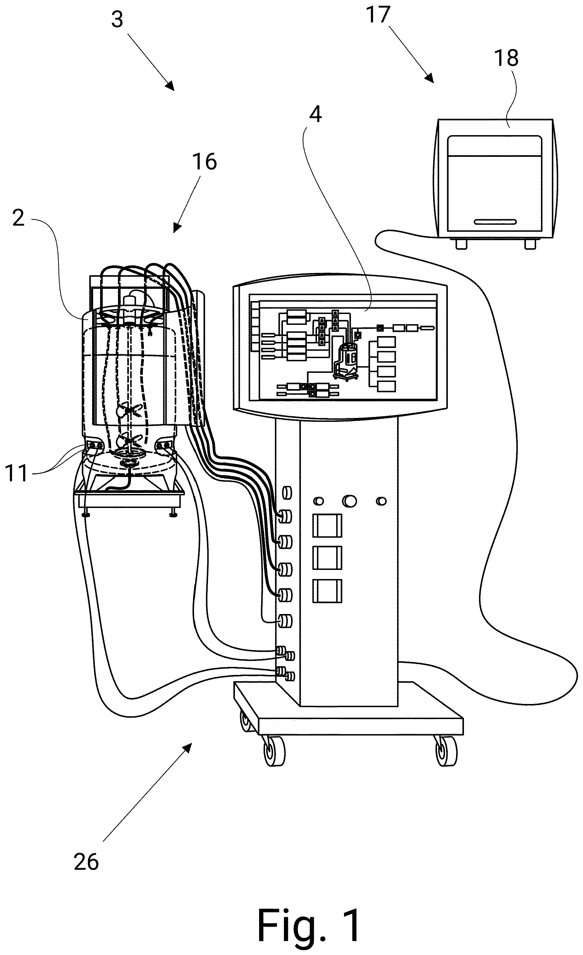

Proposed is a method for controlling a bioprocess 1. The bioprocess 1 runs in a biocontainer, here and preferably a bioreactor 2, of a bioprocess arrangement 3. The biocontainer may for example be a Flexsafe STRO bag. Fig. 1 shows an exemplary bioreactor 2 as part of a bioprocess arrangement 3. The bioprocess arrangement 3 comprises a bioprocess control unit 4, e.g. a Sartorius Biobrain^{®}. The bioprocess control unit 4 may be a single device bioprocess control unit 4 like the Biobrain^{®}. However, the bioprocess control unit 4 may also comprise multiple components, e.g. a Biobrain^{®} and a supervisory control (SCADA) system like the Sartorius BioPAT^{®} MFCS, a distributed control system (DCS) or a partially or fully cloud based system.

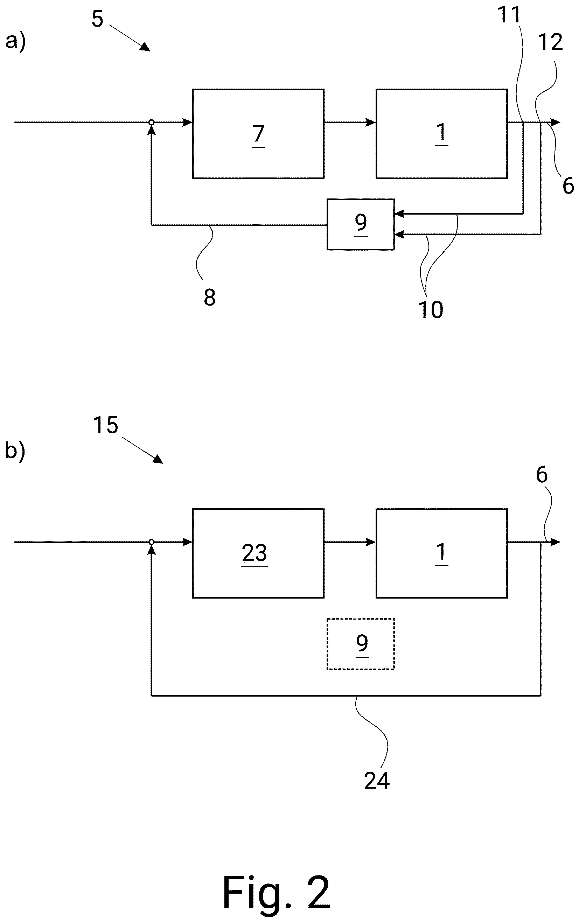

In a model control routine 5 the bioprocess control unit 4 controls at least one process parameter 6 of the bioprocess 1 according to a model-based control loop 7 (Fig. 2). Here and preferably, the process parameter 6 is a critical process parameter 6 or critical quality attribute. A model-based control loop 7 is shown in Fig. 2 a). At least one model input parameter 8 of the model-based control loop 7 is derived from a model prediction 9. The model prediction 9 is based on a model. The model input parameter 8 may be derived, and therefore the model prediction may be computed, by the bioprocess control unit 4.

At least one input parameter 10 of the model prediction 9 may be a measurement value of an online sensor 11 or atline sensor 12, as shown in Fig. 2 a). The model input parameter 8 derived from the model prediction 9 is fed into the model-based control loop 7 which outputs a system input for the bioprocess 1.

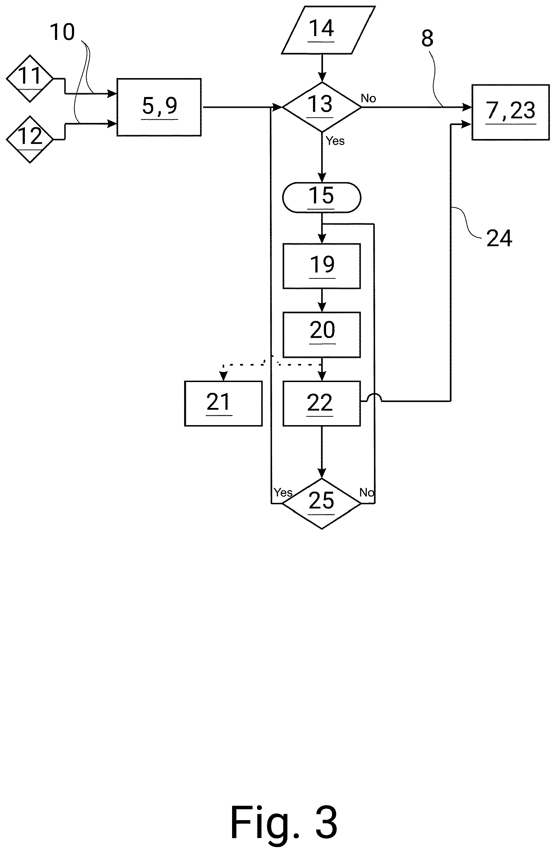

Essential and exemplarily shown in Fig. 3) is that during the model control routine 5 the bioprocess control unit 4 repeatedly performs a first evaluation 13 of the model-based control loop 7 thereby checking whether a first decision criterion is fulfilled. Fig. 3 shows at the top left side input parameters 10 of the model prediction 9 stemming from an exemplary atline sensor 12 and an exemplary online sensor 11. Behind the model prediction 9 the first evaluation 13 is shown as a check whether the first decision criterion is fulfilled. If not, the output of the model prediction 9 is fed into the model-based control loop 7. The first evaluation 13 may be based on statistical data 14 of the model prediction 9 to vet whether the model is still operating inside boundaries that promise a high accuracy. The first evaluation 13 is an evaluation of the model-based control loop 7 which, here and preferably, indirectly evaluates the model-based control loop 7 by evaluating the input of the model-based control loop 7, i.e. the model input parameter 8 being the output of the model prediction. The model input parameter 8 is evaluated by evaluating the model prediction 9. If the model prediction 9 is working with an increased uncertainty or a decreased precision, the model input parameter 8 has an increased uncertainty or decreased precision and therefore, the model-based control loop 7 using the model input parameter 8 has an increased uncertainty or decreased precision. Here and preferably, the model is validated for certain conditions of the bioprocess like certain model input parameters 8 and deemed not useable if the bioprocess leaves the boundaries of these conditions.

However, if the first decision criterion is fulfilled, the bioprocess control unit 4 switches from the model control routine 5 to a sample control routine 15. For this case, the bioprocess arrangement 3 comprises a sampling arrangement 16 for drawing a sample from the biocontainer. The term "biocontainer" relates to any fluidic structure and the like containing substances, in particular liquids, related to the bioprocess. The sample may be drawn from the bioreactor 2, a tube or any other component. The bioprocess arrangement 3 further comprises an analysis arrangement 17, which analysis arrangement 17 comprises a, in particular atline, analysis device 18. In the sample control routine 15 the analysis device 18 analyses samples provided by the sampling arrangement 16 from the biocontainer to determine a measurement value. Fig. 3 shows a step of drawing a sample 19, an optional step of aliquoting the sample 20 and of storing the aliquot 21 and a step of analysing the sample 22. The term "atline" refers to the analysis device 18 using a sample which is removed and isolated from the bioprocess. The term atline is used in accordance with ASTM E2363-06a based on ICH Q7a where online refers to a measurement where a sample is diverted from and possibly returned to a process stream, atline refers to a measurement where a sample is removed and isolated from a process stream and analyzed in close proximity to the process stream and offline refers to a measurement where a sample is removed and isolated and analyzed in a remote area. The close proximity here is used location- or time-wise, i.e. a measurement quick enough to be used for the control of the bioprocess 1 is atline if it is not online.

In the sample control routine 15 the bioprocess control unit 4 controls the bioprocess 1 according to a sample-based control loop 23. At least one sample input parameter 24 of the sample-based control loop 23 is derived from the measurement value, in particular is the measurement value. In Fig. 3, the sample input parameter 24 is input into the sample-based control loop 23 which may be identical to the model-based control loop 7 except for the source of the input. Fig. 2 b) shows the sample-based control loop 23 in comparison to the model-based control loop 7 with the model prediction 9 not being used in Fig. 2 b).

The measurement value and/or the sample input parameter 24 may correlate with, in particular be, the model input parameter 8 and/or the process parameter 6 controlled according to the model-based control loop 7. Preferably, the measurement value and/or the sample input parameter 24 and/or the model input parameter 8 and/or the process parameter 6 is a critical process parameter 6 or a critical quality attribute or a key performance indicator of the bioprocess 1.

Here and preferably during the first evaluation 13 the bioprocess control unit 4 evaluates an applicability of the model prediction 9, in particular by determining a predictivity indicator. The first decision criterion may be fulfilled if the applicability, in particular the predictivity indicator, lies below a first threshold value. It is important to note that the predictivity indicator or the applicability in general is "below" a threshold if the applicability is low, irrespective of what scale is used for the applicability and irrespective of whether the highest applicability has the highest or lowest value on that scale. It may therefore be the case that the value of the predictivity indicator falls below the first threshold value if its value rises. "Below" is therefore to be understood as a qualitative assessment, not a quantitative assessment. In the case that the model is not predicting reality with a high enough certainty, precision, or the like, the model is deemed not applicable. Other evaluations of the model are equally preferred.

It is possible that the sample-based control loop 23 is able to keep the bioprocess 1 on track if the model has lost its applicability e.g. due to lack of model training data during a specific process phase and the process gets back to a state where model training data is again sufficient or that the sample-based control loop 23 successfully navigates the bioprocess 1 through a critical phase. Here and preferably, in the sample control routine 15 the bioprocess control unit 4 repeatedly performs a second evaluation 25 of the model-based control loop 7 thereby checking whether a second decision criterion is fulfilled. If the second decision criterion is fulfilled, the bioprocess control unit 4 may switch from the sample control routine 15 back to the model control routine 5 (also shown in Fig. 3). If the model prediction 9 is working well again, there is no need to keep sampling and the model prediction 9 can be used again.

According to one embodiment it is proposed, that during the second evaluation 25 the bioprocess control unit 4 evaluates an applicability of the model, in particular by determining the predictivity indicator. The second decision criterion may be fulfilled if the applicability, in particular the predictivity indicator, lies above a second threshold value. Preferably, the second threshold value is equal to or greater than the first threshold value such that a hysteresis forms and a chance of constantly switching back and forth is reduced. It may be the case that several second evaluations 25, for example three, need to lead to an applicability above the second threshold value to switch back to the model-based control loop 7.

One reason for a model with less applicability may be that a sensor of the bioprocess arrangement 3 measuring the input parameters 10 of the model prediction 9 started drifting. Therefore, here and preferably, during the sample-based control loop 23 the bioprocess control unit 4 uses the measurement value to calibrate and/or adjust a sensor sensing an input parameter 10 of the model-based control loop 7.

According to one embodiment it is proposed, that the bioprocess control unit 4 performs the first evaluation 13 regularly time-based, for example every 15 minutes or every 2 seconds. It may additionally or alternatively be the case that the bioprocess control unit 4 performs the second evaluation 25 at least between each drawing of a sample. This may reduce the overall number of samples drawn. Further, the first evaluation 13 and the second evaluation 25 may evaluate the model regarding the same criteria, in particular may be identical.

Here and preferably, the bioprocess control unit 4 performs the first evaluation 13 event-based. Preferably, the events triggering the first evaluation 13 comprise the bioprocess 1 reaching a predefined stage and/or a sensor value of an online sensor 11 or inline sensor reaching a predefined threshold. For example, a temperature range in which the model is known to work well may be defined and if the temperature range is left the bioprocess 1 may still be within normal bounds but the sample-based control loop 23 may be used to make sure. In one embodiment, the first evaluation 13 and/or the first decision criterion varies depending on the event. In a critical stage of the bioprocess 1 for example, the first threshold value may be higher than in a less critical stage. This may lead to more false positive cases of switching into the sample control routine 15 but less bioprocesses 1 running out of bounds without the sample-based control loop 23 being able to save the bioproduct.

According to one embodiment it is proposed, that a bioproduct produced between the determination that the first decision criterion is fulfilled and the receipt of a first measurement value by the bioprocess control unit 4 is flagged with a risk flag. Preferably, the risk flag is removed based on an assessment of the model based on the measurement value by the bioprocess control unit 4. It may be the case that all bioproduct needs to be discarded or sent into a further quality control if the measurement shows that the bioprocess 1 had left the specified range.

Here and preferably, the sampling arrangement 16 automatically draws the sample from the biocontainer, and/or, that the bioprocess arrangement 3 comprises a sample preparation arrangement which prepares and/or aliquots the sample. The bioprocess arrangement 3 may comprise a routing arrangement 26 which automatically transfers the sample from the sample arrangement 16 to the analysis device 18 and/or the sample preparation arrangement. Here and preferably, the bioprocess arrangement 3 fully automatically performs all steps from sampling to analysis of the sample.

The optionally aliquoted sample may be stored for further references, duplicates, and optional analysis by an offline master reference method.

In one embodiment, the first evaluation 13 comprises, at least sometimes, drawing and analyzing a sample and evaluating the model based on this analysis. In this case, this sample-based first evaluation 13 is performed less often than a sampling for a sample-based control would be performed. In another embodiment, the first evaluation 13 is not sample-based.

According to one embodiment it is proposed, that the model of the model-based control loop 7 uses at least one inline sensor and/or at least one online sensor 11 as a model input parameter 8.

It may also be the case that during the model control routine 5 the bioprocess control unit 4 controls several process parameters 6 according to several control loops, at least two of the several control loops being model-based control loops 7 controlling model process parameters 6, that the bioprocess control unit 4 in the sample control routine 15 controls the model process parameters 6 according to sample-based control loops 23 and preferably other process parameters 6 according to the same control loops as in the model control routine 5.

Another teaching which is of equal importance relates to a bioprocess control unit 4 adapted for use in the proposed method.

Another teaching which is of equal importance relates to a bioprocess arrangement 3 adapted for use in the proposed method, comprising a proposed bioprocess control unit 4.

## Claims

1. Method for controlling a bioprocess (1), wherein the bioprocess (1) runs in a biocontainer of a bioprocess arrangement (3), wherein the bioprocess arrangement (3) comprises a bioprocess control unit (4), wherein in a model control routine (5) the bioprocess control unit (4) controls at least one process parameter (6) of the bioprocess (1) according to a model-based control loop (7), wherein at least one model input parameter (8) of the model-based control loop (7) is derived from a model prediction (9),
**characterized in**
**that** during the model control routine (5) the bioprocess control unit (4) repeatedly performs a first evaluation (13) of the model-based control loop (7) thereby checking whether a first decision criterion is fulfilled, that if the first decision criterion is fulfilled, the bioprocess control unit (4) switches from the model control routine (5) to a sample control routine (15),
**that** the bioprocess arrangement (3) comprises a sampling arrangement (16) for drawing a sample from the biocontainer, that the bioprocess arrangement (3) comprises an analysis arrangement (17), that the analysis arrangement (17) comprises a, in particular atline, analysis device (18),
**that** in the sample control routine (15) the analysis device (18) analyses samples provided by the sampling arrangement (16) from the biocontainer to determine a measurement value, that in the sample control routine (15) the bioprocess control unit (4) controls the bioprocess (1) according to a sample-based control loop (23), that at least one sample input parameter (24) of the sample-based control loop (23) is derived from the measurement value.

2. Method according to claim 1, **characterized in that** the measurement value and/or the sample input parameter (24) correlates with, in particular is, the model input parameter (8) and/or the process parameter (6) controlled according to the model-based control loop (7), preferably, that the measurement value and/or the sample input parameter (24) and/or the model input parameter (8) and/or the process parameter (6) is a critical process parameter (6) or a critical quality attribute or a key performance indicator of the bioprocess (1).

3. Method according to claim 1 or 2, **characterized in that** during the first evaluation (13) the bioprocess control unit (4) evaluates an applicability of the model prediction (9), in particular by determining a predictivity indicator, that the first decision criterion is fulfilled if the applicability, in particular the predictivity indicator, lies below a first threshold value.

4. Method according to one of the preceding claims, **characterized in that** in the sample control routine (15) the bioprocess control unit (4) repeatedly performs a second evaluation (25) of the model-based control loop (7) thereby checking whether a second decision criterion is fulfilled, that if the second decision criterion is fulfilled, the bioprocess control unit (4) switches from the sample control routine (15) back to the model control routine (5).

5. Method according to claim 4, **characterized in that** during the second evaluation (25) the bioprocess control unit (4) evaluates an applicability of the model, in particular by determining the predictivity indicator, that the second decision criterion is fulfilled if the applicability, in particular the predictivity indicator, lies above a second threshold value, preferably, that the second threshold value is equal to or greater than the first threshold value.

6. Method according to one of the preceding claims, **characterized in that** during the sample-based control loop (23) the bioprocess control unit (4) uses the measurement value to calibrate and/or adjust a sensor sensing an input parameter (10) of the model-based control loop (7).

7. Method according to one of the preceding claims, **characterized in that** the bioprocess control unit (4) performs the first evaluation (13) regularly time-based, and/or, that the bioprocess control unit (4) performs the second evaluation (25) at least between each drawing of a sample, and/or, that the first evaluation (13) and the second evaluation (25) evaluate the model regarding the same criteria, in particular are identical.

8. Method according to one of the preceding claims, **characterized in that** the bioprocess control unit (4) performs the first evaluation (13) event-based, preferably, that the events triggering the first evaluation (13) comprise the bioprocess (1) reaching a predefined stage and/or a sensor value of an online sensor (11) or inline sensor reaching a predefined threshold, and/or, that the first evaluation (13) and/or the first decision criterion varies depending on the event.

9. Method according to one of the preceding claims, **characterized in that** a bioproduct produced between the determination that the first decision criterion is fulfilled and the receipt of a first measurement value by the bioprocess control unit (4) is flagged with a risk flag, preferably, that the risk flag is removed based on an assessment of the model based on the measurement value by the bioprocess control unit (4).

10. Method according to one of the preceding claims, **characterized in that** the sampling arrangement (16) automatically draws the sample from the biocontainer, and/or, that the bioprocess arrangement (3) comprises a sample preparation arrangement which prepares and/or aliquots the sample, and/or, that the bioprocess arrangement (3) comprises a routing arrangement (26) which automatically transfers the sample from the sample arrangement (16) to the analysis device (18) and/or the sample preparation arrangement, preferably, that the bioprocess arrangement (3) fully automatically performs all steps from sampling to analysis of the sample.

11. Method according to one of the preceding claims, **characterized in that** the model of the model-based control loop (7) uses at least one inline sensor and/or at least one online sensor (11) as a model input parameter (8).

12. Method according to one of the preceding claims, **characterized in that** during the model control routine (5) the bioprocess control unit (4) controls several process parameters (6) according to several control loops, at least two of the several control loops being model-based control loops (7) controlling model process parameters (6), that the bioprocess control unit (4) in the sample control routine (15) controls the model process parameters (6) according to sample-based control loops (23) and preferably other process parameters (6) according to the same control loops as in the model control routine (5).

13. Bioprocess control unit adapted for use in the method according to one of the preceding claims.

14. Bioprocess arrangement adapted for use in the method according to one of claims 1 to 12, comprising a bioprocess control unit (4) according to claim 13.
